Europäisches Patentamt

European Patent Office

Office européen des brevets

(11) Veröffentlichungsnummer: **0 375 920**
**A1**

# (12) EUROPÄISCHE PATENTANMELDUNG

(21) Anmeldenummer: **89121381.1**

(22) Anmeldetag: **18.11.89**

(51) Int. Cl.5: **C07C 17/22, C07C 19/02, C07C 17/33**

(30) Priorität: **30.11.88 DE 3840340**

(43) Veröffentlichungstag der Anmeldung:
**04.07.90 Patentblatt 90/27**

(84) Benannte Vertragsstaaten:
**BE DE FR GB IT NL**

(71) Anmelder: **BASF Aktiengesellschaft
Carl-Bosch-Strasse 38
D-6700 Ludwigshafen(DE)**

(72) Erfinder: **Widder, Rudi, Dr.
In der Taesch 7
D-6906 Leimen(DE)**
Erfinder: **Decker, Martin, Dr.
Maria-Stuart-Strasse 21
D-6700 Ludwigshafen(DE)**

(54) **Verfahren zur Herstellung von gegebenenfalls substituierten Alkyl- oder Alkenylchloriden.**

(57) Verfahren zur Herstellung von gegebenenfalls substituierten Alkyl- oder Alkenylchloriden durch Decarboxylierung von entsprechenden Alkyl- oder Alkenylchlorformiaten bei erhöhten Temperaturen, indem man die Decarboxylierung in Gegenwart einer katalytischen Menge eines quartären Ammonium- oder Phosphoniumsalzes oder eines ternären Sulfoniumsalzes vornimmt.

EP 0 375 920 A1

# Verfahren zur Herstellung von gegebenenfalls substituierten Alkyl- oder Alkenylchloriden

Die vorliegende Erfindung betrifft ein Verfahren zur Herstellung von gegebenenfalls substituierten Alkyl- oder Alkenylchloriden durch Decarboxylierung von entsprechenden Alkyl- oder Alkenylchlorformiaten in Gegenwart eines quartären Ammonium- oder Phosphoniumsalzes oder eines ternären Sulfoniumsalzes.

Aus J. Chem. Soc., Sec. B, 747-751 (1971), ist bekannt, daß man gesättigte Alkylchloride aus gesättigten Alkylchlorformiaten bei erhöhten Temperaturen in Gegenwart von Pyridiniumchlorid erhält. Die Reinigung erfolgt anschließend durch Destillation.

Aus der DE-A-25 45 659 ist bekannt, daß Alkylchloride in aprotischen Lösungsmitteln bei 80 bis 200°C durch Decarboxylierung von Alkylchlorformiaten erhalten werden. Das Lösungsmittel muß anschließend destillativ entfernt werden und bei der technischen Durchführung entstehen unerwünschte Salzablagerungen.

Ferner ist aus der DE-A-29 31 777 bekannt, daß man Alkylchlorformiate bei 90 bis 170°C in Gegenwart eines Trialkylamin-Hydrochlorides zu Alkylchloriden decarboxylieren kann. Die Abtrennung des Alkylchlorids vom Katalysator erfolgt durch Destillation, der gegebenenfalls ein Waschvorgang mit Wasser vorgeschaltet ist.

Der vorliegenden Erfindung lag daher die Aufgabe zugrunde, einen besseren Zugang zu den Alkylchloriden zu finden und den Nachteilen der bekannten Verfahren abzuhelfen.

Demgemäß wurde ein neues und verbessertes Verfahren zur Herstellung von gegebenenfalls substituierten Alkylchloriden durch Decarboxylierung von entsprechenden Alkylchlorformiaten bei erhöhten Temperaturen gefunden, welches dadurch gekennzeichnet ist, daß man die Decarboxylierung in Gegenwart einer katalytischen Menge eines quartären Ammonium- oder Phosphoniumsalzes oder eines ternären Sulfoniumsalzes vornimmt.

Das erfindungsgemäße Verfahren kann so durchgeführt werden, daß das gegebenenfalls substituierte Alkyl- oder Alkenylchlorformiat und das quartäre Ammonium- oder Phosphoniumsalz oder das ternäre Sulfoniumsalz zusammengefügt werden und die Reaktionsmischung anschließend auf 50 bis 200°C erhitzt wird. Eine verfahrenstechnisch bevorzugte Ausführungsform besteht darin, den Katalysator in dem Alkyl- oder Alkenylchlorid vorzulegen, das bei der Reaktionstemperatur von 50 bis 200°C aus dem gleichmäßig zudosierten Alkyl- oder Alkenylchlorformiat entsteht. Diese Verfahrensvarianten lassen sich diskontinuierlich oder vorzugsweise kontinuierlich durchführen.

Bei der diskontinuierlichen Verfahrensweise legt man einen Teil des herzustellenden Alkyl- oder Alkenylchlorids und den Katalysator vor, erhitzt auf die gewünschte Reaktionstemperatur und gibt das Alkyl- oder Alkenylchlorformiat gleichmäßig hinzu. Der Katalysator kann hierbei als feste Substanz oder als wässrige Lösung eingesetzt werden. Das mit dem Katalysator eingebrachte Wassser sollte möglichst gering sein und aus der Reaktionsmischung wieder entfernt werden.

Zur kontinuierlichen Durchführung des Verfahrens führt man dem mit Alkyl- oder Alkenylchlorid und Katalysator gefüllten Reaktorsystem das Alkyl- oder Alkenylchlorformiat und die Katalysatorlösung bei der geeigneten Reaktionstemperatur zu, kondensiert aus der Gasphase das mitgeführte Alkyl- oder Alkenylchlorid aus, während am Ausgang des Reaktorsystems gegebenenfalls flüssiges Reaktionsprodukt entnommen wird. Als Katalysator verwendet man möglichst konzentrierte wässrige Lösungen und entfernt das Wasser aus der Reaktionszone. Vorzugsweise verwendet man jedoch eine Lösung des Katalysators in dem herzustellenden Alkyl- oder Alkenylchlorid.

Die erforderliche Katalysatormenge liegt im Bereich von 0,001 bis 5 Mol% des umzusetzenden Alkyl- oder Alkenylchlorformiats. Vorteilhaft verwendet man eine Menge von 0,01 bis 3 Mol% und besonders bevorzugt setzt man eine Menge von 0,1 bis 1,5 Mol% ein. Sofern die Alkyl- oder Alkenylchloride bei der Reaktionstemperatur weitgehend gasförmig das Reaktionssystem verlassen, ist der untere Bereich der genannten Katalysatormenge geeignet.

In aller Regel arbeitet man bei Temperaturen von 90 bis 170°C, vorzugsweise bei 110 bis 150°C, insbesondere bei 125 bis 140°C.

Als Katalysatoren eignen sich besonders quartäre Ammonium- und Phosphoniumsalze der allgemeinen Formel

$$(R_4A)^{\oplus}X^{\ominus}$$

in der A für Stickstoff oder Phospor, X für Halogen wie Fluor, Chlor, Brom oder Jod, Sulfat und Phosphat und R für unverzweigtes oder verzweigtes Alkyl, bevorzugt $C_1$- bis $C_{20}$-Alkyl, Aryl wie Phenyl oder Benzyl steht, wobei die vier Reste R in der obigen Formel gleich oder verschieden sein können. Diese Katalysatoren eignen sich als Phasentransferkatalysatoren. Als Beispiele seien folgende Verbindungen genannt:
Tetramethylammoniumchlorid, Tetraethylammoniumchlorid, Tetrabutylammoniumbromid, Benzyl-trimethylammoniumchlorid, Tetrabutylphosphoniumbromid, Tetraphenylphosphoniumchlorid und Tributylsulfoniumchlorid.

Die vorgelegte Menge des Katalysators ist im allgemeinen unkritisch, da sie im Reaktionsraum verbleibt. Bei der Herstellung schwerflüchtiger Alkyl- oder Alkenylchloride wählt man eine Katalysatormenge von 0,05 bis 1 Mol%, vorzugsweise 0,1 bis 0,5 Mol%.

Die Alkyl- oder Alkenylchlorformiate werden in an sich bekannter Weise (Houben/Weyl, Band E4 (1983), Seite 15 ff.) durch Umsetzung der entsprechenden Alkohole mit Phosgen hergestellt.

Man kann anstelle der Alkyl- oder Alkenylchlorformiate auch die entsprechenden Alkohole einsetzen und zum Reaktionsgemisch Phosgen geben. Es werden dann zunächst die Alkyl- oder Alkenylchlorformiate gebildet, die dann sofort unter Decarboxylierung zu den Alkyl- oder Alkenylchloriden weiterreagieren.

Die als Ausgangsstoffe verwendeten Alkyl- oder Alkenylchlorformiate können gegebenenfalls durch Alkoxy, Halogen, Aryl, Chlorformiat (-OCOCl) oder Chlorformiatalkoxy substituiert sein. Beispiele für diese Substitutionen sind Methoxy, Ethoxy, n-Propoxy, iso-Propoxy, n-Butoxy, iso-Butoxy, sec.-Butoxy, tert.-Butoxy, Pentoxy, Hexoxy, Octoxy, Fluor, Chlor, Brom, Phenyl, -OCOCl, -OCH$_2$OCOCl, -O(CH$_2$)$_2$OCOCl, -O(CH$_2$)$_3$OCOCl, -O(CH$_2$)$_4$OCOCl und -O(CH$_2$)$_6$OCOCl. Unter den Alkylchlorformiaten sind solche bevorzugt, die im Alkylteil 3 bis 20 C-Atome, besonders bevorzugt 3 bis 10 C-Atome enthalten, wie n-Propyl, iso-Propyl, n-Butyl, iso-Butyl, sec.-Butyl, tert.-Butyl, Pentyl, Hexyl, Heptyl, Octyl, Nonyl und Decyl. Unter den Alkenylchlorformiaten sind solche bevorzugt, die im Alkenylteil 3 bis 20 C-Atome, besonders bevorzugt 3 bis 10 C-Atome enthalten, wie n-Propenyl, iso-Propenyl, n-Butenyl, iso-Butenyl, sec.-Butenyl, tert.-Butenyl, Pentenyl, Hexenyl, Heptenyl, Octenyl, Nonenyl und Decenyl.

Nach dem erfindungsgemäßen Verfahren wird z.B. 1,6-Hexandiol-bis-chlorformiat in Gegenwart katalytischer Mengen Tetrabutylammoniumbromid zu rohem, katalysatorhaltigem 1,6-Dichlorhexan decarboxyliert und dieses direkt mit Natriumcyanid unter erneuter Katalyse durch Tetrabutylammoniumbromid zu Korksäuredinitril umgesetzt.

Die gegebenenfalls substituierten Alkylchloride eignen sich als Zwischenprodukte für die Herstellung von Bacterciden, Pflanzenschutzmittel, Pharmazeutika und für Zwischenprodukte mit anderen funktionellen Gruppen.

## Beispiele

### Beispiel 1

In einem Rührbehälter mit 1.000 l werden 107 kg 1,6-Dichlorhexan und 2,7 kg einer 50%igen wäßrigen Lösung von Tetrabutylammoniumbromid vorgelegt und auf 130°C erhitzt. Dann werden je Stunde 200 kg 1,6-Hexandiol-bis-chlorformiat bei 130 bis 135°C gleichmäßig eindosiert. Insgesamt werden 450 kg 1,6-Hexandiol-bis-chlorformiat zur Reaktion gebracht. Nach Zulaufende wird die Reaktionsmischung noch 30 Minuten bei 130°C gehalten und danach auf Raumtemperatur abgekühlt.

Es werden 393 kg (98,8%) rohes 1,6-Dichlorhexan mit einer Reinheit von 98,2 % erhalten, ohne Berücksichtigung des als Lösungsmittel eingesetzten 1,6-Dichlorhexans.

### Beispiel 2

In der gleichen Apparatur wie im Beispiel 1 werden 50 kg 1,6-Dichlorhexan und 0,6 kg reines Tetrabutylammoniumbromid vorgelegt und auf 130°C erhitzt.

Es werden danach stündlich 100 kg 1,6-Hexandiol-bis-chlorformiat bei 130 bis 135°C eindosiert und nach Zulaufende die Temperatur noch 1 Stunde bei 130°C gehalten. Insgesamt werden 300 kg 1,6-Hexandiol-bis-chlorformiat umgesetzt.

Nach dem Abkühlen auf Raumtemperatur werden 241 kg (96,5 %) rohes 1,6-Dichlorhexan erhalten, ohne Berücksichtigung des als Lösungsmittel eingesetzten 1,6-Dichlorhexans. Die Reinheit beträgt 97,3 %.

### Beispiel 3

In der Apparatur des Beispiels 1 werden 130 kg 1,6-Dichlorhexan und 8,7 kg Benzyl-dimethyl-laurylammoniumchlorid vorgelegt und auf 130°C erhitzt. Bei 130°C werden je Stunde 136 kg 1,6-Hexandiol-bis-chlorformiat gleichmäßig zugegeben. Insgesamt werden 477 kg 1,6-Hexandiol-bis-chlorformiat umgesetzt.

Nach der Beendigung des Zulaufs wird noch 1,5 Stunden bei 130°C nachbehandelt und dann auf Raumtemperatur abgekühlt. Es werden 442 kg (96,1 %) Rohprodukt 1,6-Dichlorhexan erhalten, ohne Berücksichtigung des als Lösungsmittel eingesetzten 1,6-Dichlorhexans. Die Reinheit beträgt 95,7 %.

## Ansprüche

1. Verfahren zur Herstellung von gegebenenfalls substituierten Alkyl-oder Alkenylchloriden durch Decarboxylierung von entsprechenden Alkyl- oder Alkenylchlorformiaten bei erhöhten Temperaturen, dadurch gekennzeichnet, daß man die Decarboxylierung in Gegenwart einer katalytischen Menge eines quartären Ammonium- oder Phosphoniumsalzes oder eines ternären Sulfoniumsalzes vornimmt.

2. Verfahren nach Anspruch 1, dadurch gekennzeichnet, daß man, bezogen auf die Alkyl- oder Alkenylchlorformiate, 0,001 bis 5 Mol% eines quartären Ammonium- oder Phosphoniumsalzes oder eines ternären Sulfoniumsalzes anwendet.

3. Verfahren nach Anspruch 1, dadurch gekennzeichnet, daß man eine 5 bis 80 %ige wäßrige Lösung eines quartären Ammonium- oder Phosphoniumsalzes oder eines ternären Sulfoniumsalzes verwendet.

4. Verfahren nach Anspruch 1, dadurch gekennzeichnet, daß man die Decarboxylierung bei 50 bis 200 °C durchführt.

5. Verfahren nach Anspruch 1, dadurch gekennzeichnet, daß man als Katalysator Tetramethylammoniumchlorid, Tetraethylammoniumchlorid, Tetrabutylammoniumbromid, Benzyl-trimethylammoniumchlorid, Tetrabutylphosphoniumbromid, Tetraphenylphosphoniumchlorid und/oder Tributylsulfoniumchlorid verwendet.

6. Verfahren nach Anspruch 1, dadurch gekennzeichnet, daß man anstelle der Alkyl- oder Alkenylchlorformiate ein Reaktionsgemisch einsetzt, das durch Umsetzung der entsprechenden Alkohole mit Phosgen erhalten wurde.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | US-A-4 814 524 (R.G. BRIODY)<br>* Ansprüche 1-10; Spalte 1, Zeilen 23-27 *<br>--- | 1-6 | C 07 C 17/22<br>C 07 C 19/02<br>C 07 C 17/33 |
| D,A | EP-A-0 025 829 (BASF AG)<br>* Anspruch 1 *; & DE - A - 2931777 (Kat. D)<br>--- | 1 | |
| D,A | CHEMICAL ABSTRACTS<br>Band 74, Nr. 25, 21.Juni 1971, Seite 498, Zusammenfassung Nr. 140621f, Columbus, Ohio, US; H. HUDSON et al.: "Factors in the formation of isomerically and optically pure alkyl halides. VII. Rearrangements occuring during the thermal decompositon of alkyl chloroformates"; & J. Chem. Soc. B 1971 (4) 747-51<br>--- | 1 | |
| A | CHEMICAL ABSTRACTS<br>Band 67, Nr. 17, 23. Oktober 1967, Seite 7701, Zusammenfassung Nr. 81828j, Columbus, Ohio, USA; A.B. FOSTER et al.: "Pyridine-catalyzed decarboxylation of cis- and trans-4-tert-butylcyclohexyl chloroformate" & Carbohyd. Res. 4(4) 352-4, 1967<br>--- | 1 | RECHERCHIERTE SACHGEBIETE (Int. Cl.5)<br><br>C 07 C 17/00<br>C 07 C 19/00 |
| D,A | DE-A-2 545 659 (BASF)<br>* Anspruch 1 *<br>--- | 1 | |
| A | EP-A-0 118 241 (ICI)<br>* Seite 2, Zeilen 5-23 *<br>----- | 1 | |

Der vorliegende Recherchenbericht wurde für alle Patentansprüche erstellt

| Recherchenort | Abschlußdatum der Recherche | Prüfer |
|---|---|---|
| BERLIN | 22-02-1990 | PROBERT C.L. |